# EUROPEAN PATENT SPECIFICATION

(11) **EP 2 777 654 B1**
(45) Date of publication and mention of the grant of the patent: **17.04.2019**
(21) Application number: 14275069.4
(22) Date of filing: 14.03.2014
(51) Int. Cl.: A61F 2/966

(54) **Wireless medical device release mechanism**
Drahtloser Auslösemechanismus für ein Medizinprodukt
Dispositif médical mécanisme de libération sans fil

(30) Priority: 15.03.2013 US 201313835877
(43) Date of publication of application: 17.09.2014
(73) Proprietor: Cook Medical Technologies LLC, Bloomington, IN 47404 (US)
(72) Inventor: Harvel, William J., West Lafayette, IN 47906 (US); Hadley, Richard S., Otterbein, IN 47970 (US); Huser, Matthew S., Lafayette, IN 47909 (US)
(74) Representative: Williams Powell

(56) References cited:
- JP-A- 2000 262 632
- US-A1- 2008 033 528
- US-A1- 2008 221 703
- US-A1- 2008 300 616
- US-A1- 2009 270 966
- US-A1- 2011 190 865
- US-A1- 2012 053 671
- US-A1- 2012 116 413

## Description

### FIELD OF APPLICATION

This application relates to a medical device delivery system. More particularly, the application relates to a stent/stent-graft delivery system that allows a portion of a self-expanding stent/stent-graft to be released without the use of a trigger wire.

### BACKGROUND

Stents may be inserted into an anatomical vessel or duct for various purposes. Stents may maintain or restore patency in a formerly blocked or constricted passageway, for example, following a balloon angioplasty procedure. Other stents may be used for different procedures, for example, stents placed in or about a graft have been used to hold the graft in an open configuration to treat an aneurysm. Additionally, stents coupled to one or both ends of a graft may extend proximally or distally away from the graft to engage a healthy portion of a vessel wall away from a diseased portion of an aneurysm to provide endovascular graft fixation.

Stents may be either self-expanding or balloon-expandable, or they can have characteristics of both types of stents. Self-expanding stents may be delivered to a target site in a compressed configuration and subsequently expanded by removing a delivery sheath, removing trigger wires and/or releasing diameter reducing ties. With self-expanding stents, the stents expand primarily based on their own expansive force without the need for further mechanical expansion. In a stent made of a shape-memory alloy such as nitinol, the shape-memory alloy may be employed to cause the stent to return to a predetermined configuration upon removal of the sheath or other device maintaining the stent in its predeployment configuration.

When trigger wires are used as a deployment control mechanism, the trigger wires may releasably couple the proximal and/or distal ends of a stent or stent-graft to a delivery catheter. Typically, one or more trigger wires are looped through a portion of the stent near a vertex of the stent. For example, trigger wires may be used to restrain a "Z-stent" or Gianturco stent comprising a series of substantially straight segments interconnected by a series of bent segments. The trigger wires may be disposed through, and pull upon, the bent segments to pull the stent closely against the delivery catheter.

Trigger wires also may be used in conjunction with different stent designs, such as cannula-cut stents having relatively acute or pointed bends. The designs of cannula-cut stents may facilitate compression of the stent to a relatively small delivery profile due to the tight bends of the apices. With such stents, the trigger wires may be looped around one or more vertices formed beneath the proximal and/or distal apices, e.g., a location where an individual apex splits into two separate strut segments.

If trigger wires are threaded through the vertices of such cannula-cut stents, the trigger wires may become crimped at the vertices during compression of the stent to a reduced diameter delivery profile. If the trigger wires are crimped between the strut segments, the trigger wires and/or stent segments may become damaged during delivery, particularly for nickel-titanium stents that may be sensitive to surface imperfections. Furthermore, when compressing a cannula-cut stent having relatively acute bends to a significantly reduced radial profile, barbs disposed near the apices of the stent may become entangled with the stent struts and/or the trigger wires. Still further, in some instance, trigger wires may require a relatively high deployment force when being retracted, and the provision of multiple trigger wires may add to the profile of the delivery system.

US2012/116413 A1 discloses a medical delivery system for use with an expandable medical device having a resiliently flexible looped portion extending from a section of the expandable medical device, the system including a shaft including a proximal end and a distal end and an interposed internal lumen, the shaft having a notched portion at the proximal end of the shaft, wherein the capture element receives the looped portion of the expandable medical device for capture, and a sheath coaxially disposed over the shaft, wherein the sheath is movable from a first axial position where the sheath covers the notched portion to a second axial position where the notched portion is not covered by the sheath thereby releasing the looped portion from capture, wherein the expandable medical device is coupled to the shaft when the sheath is in the first axial position.

US 2011/190865 A1, US 2008/300616 A1, US 2008/221703 A1 and US 2012/053671 A1, US 2009/270966 A1 also disclose release arrangements for medical devices.

### SUMMARY OF THE INVENTION

The present invention seeks to provide an improved medical device delivery system.

According to the present invention, there is provided a medical device delivery system for a stent or a stent-graft as specified in claim 1.

The descriptions below include medical device delivery systems that allow the medical device, and in particular a stent/stent-graft to be released without the use of trigger wires. In one embodiment, the delivery system includes an expandable medical device having a looped portion extending from a section of the medical device, where the looped portion is resiliently flexible, a shaft comprising a proximal end and a distal end and an interposed internal lumen, the shaft having a capture element at the proximal end of the shaft, where the capture element receives the looped portion of the expandable medical device, and a sheath coaxially disposed over the shaft, where the sheath is movable from a first axial position where the sheath covers the capture element to a second axial position where the capture element is not covered by the sheath and where the expandable medical device is coupled to the shaft when the sheath is in the first axial position.

A method of releasing the medical device includes delivering an implantable expandable medical device to a desired location, where a looped portion of the implantable expandable medical device is attached to a capture element located at a proximal end of a shaft and where a sheath is coaxially disposed over the capture element, moving the sheath away from the capture element in a direction parallel to a length of the shaft, uncovering the capture element, and releasing the looped portion of the implantable expandable medical device from the capture element.

In another embodiment, the delivery system can be used with an expandable medical device having a resiliently flexible looped portion extending from a section of the expandable medical device, the system includes a shaft comprising a proximal end and a distal end and an interposed internal lumen, the shaft having a capture element at the proximal end of the shaft, where the capture element receives the looped portion of the expandable medical device, and a sheath coaxially disposed over the shaft, where the sheath is movable from a first axial position where the sheath covers the capture element to a second axial position where the capture element is not covered by the sheath and where the expandable medical device is coupled to the shaft when the sheath is in the first axial position.

Other systems, methods, features and advantages will be, or will become, apparent to one with skill in the art upon examination of the following figures and detailed description. It is intended that all such additional systems, features and advantages be included within this description, be within the scope of the invention as long as they are defined by the following claims.

### BRIEF DESCRIPTION OF THE DRAWINGS

Embodiments of the present invention are described below, by way of example only, with reference to the accompanying drawings, in which:
FIG. 1 is a perspective view of an exemplary medical device delivery system;
FIG. 2 is a perspective view of the exemplary medical device delivery system;
FIG. 3 is a side view of the exemplary medical device delivery system;
FIG. 4 is another side view of the exemplary medical device delivery system;
FIG. 5 is a side view of portions of another exemplary medical device delivery system;
FIG. 6 is a front view of portions of another exemplary medical device delivery system;
FIG. 7 is a top view of portions of another exemplary medical device delivery system;
FIG. 8 is a perspective view of portions of a second exemplary medical device delivery system;
FIG. 9 is a top view of portions of the second exemplary medical device delivery system;
FIG. 10 is a side-sectional elevation view of portions of the second exemplary medical device delivery system;
FIG. 11 is a perspective view of portions of a third exemplary medical device delivery system;
FIG. 12 is a side elevation view of portions of the third exemplary medical device delivery system;
FIG. 13 is a front view of portions of the third exemplary medical device delivery system;
FIG. 14 is a top view of portions of the third exemplary medical device delivery system;
FIG. 15 is a side view of portions of another exemplary medical device delivery system;
FIG. 16 is a side view of portions of another exemplary medical device delivery system; and
FIG. 17 is a flow diagram of a method for releasing a medical device according to another embodiment of the invention.

### DETAILED DESCRIPTION

In the present application, the term "proximal" refers to a direction that is generally closest to the heart during a medical procedure, while the term "distal" refers to a direction that is furthest from the heart during a medical procedure.

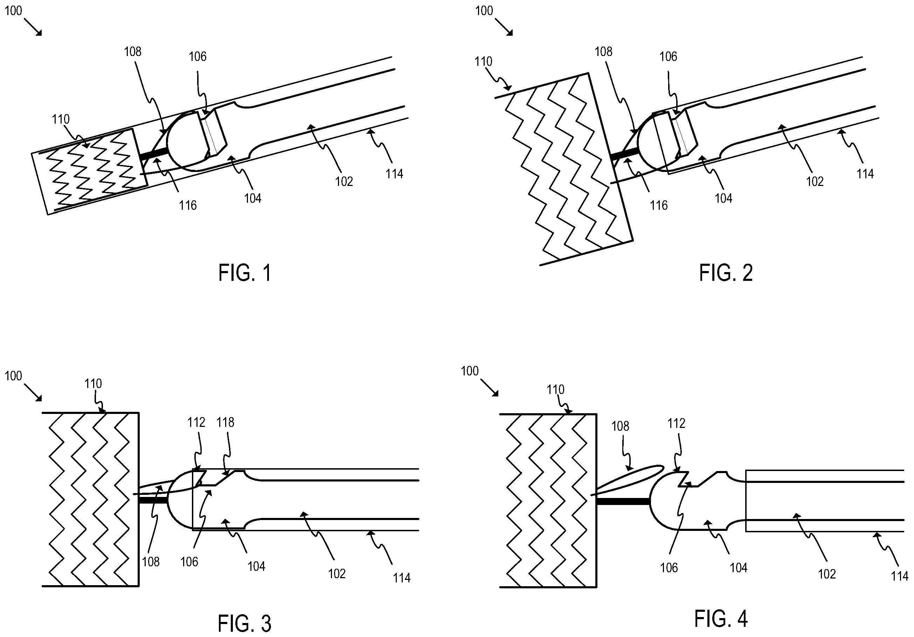

Various embodiments of the medical device delivery system are shown in Figures 1 - 17. In general, the medical device delivery system may allow for the release of a portion of a self-expanding medical device, in particular a stent/stent graft, from the delivery system without the use of release wires or trigger wires. The medical device may be delivered to a location in a body while the medical device is held in a compressed state by a sheath coaxially located over the medical device. The sheath may radially restrain the medical device. Axial movement of the sheath away from the medical device may allow the medical device to expand. The medical device may include a looped portion that is axially restrained by a capture element in a delivery tool. The medical device may be releasably attached to delivery tool while the looped portion is located in the capture element. The sheath may cover the capture element and prevent the looped portion from being removed from the capture element. The looped portion may be released from the capture element after the sheath is withdrawn from the capture element. The medical device may no longer be attached to the delivery tool after the looped portion is released from the capture element. The medical device may be fixed in its location in the body after the medical device is no longer attached to the delivery tool.

Figure 1 is a perspective view of a portion of an exemplary medical device delivery system 100. In Figure 1, a delivery tool may have a distal end (not shown), a shaft 102, and a proximal end 104. The shaft 102 and proximal end 104 may be cylindrical or any other elongated shape capable of entering a body cavity. The delivery tool may include a lumen provided through the shaft 102. The lumen may allow a guidewire or cannula 116 to pass axially through the delivery tool.

A capture element in the form of a notched portion 106 is located at the proximal end 104 of the delivery tool. The notched portion 106 may be any shape capable of allowing a looped portion 108 of a medical device 110 to enter and be held in the notched portion 106.

A medical device 110 may be located proximally from the proximal end 104 of the delivery tool. The medical device 110 may be a stent-graft or any other medical device designed to be located intravascularly or within a body cavity. The medical device 110 shown in Figure 1 may be shown in compressed state. The medical device may be held in a compressed state by a sheath 114 that is coaxially located over the medical device 110. Sheath 114 may radially restrain the medical device 110 while the sheath 114 is coaxially located over the medical device 110. Removal of sheath 114 may allow medical device 110 to expand radially, as shown in Figure 2. The medical device includes a looped portion 108 located at its distal end. The looped portion 108 may be any resilient and flexible structure, such as a suture, attached to the medical device 110. The looped portion 108 may be tied to the medical device 110 or attached in some other manner such as by rivets or by fusing the looped portion 108 to the medical device 110. The looped portion 108 may be sized to extend into the capture element 106 while the looped portion 108 is attached to the medical device 110. The looped portion 108 may be sized large enough to allow slight movement of the medical device 110 relative to the delivery tool while the looped portion 108 is located in the capture element 106.

The medical device delivery system 100 may include a sheath 114 that surrounds the medical device 110, shaft 102, and proximal end 104 of the delivery tool. In Figures 1 - 4, the sheath 114 is shown as transparent to allow objects covered by the sheath 114 to be visible. The sheath 114 may actually be opaque or transparent. The sheath 114 may move axially relative to the shaft 102, as shown in Figures 1 - 4.

Figure 2 shows the medical device 110 in an expanded state. The compressed or expanded states of medical device 110 shown in Figures 1 - 4 are for illustrative purposes only. The actual amount of compression or expansion may vary as required by the medical device and the application of the medical device delivery system. Movement of sheath 114 axially away from medical device 110, as shown in Figure 2, may allow medical device 110 to expand.

Figure 3 is a side view of the exemplary medical device delivery system 100. In Figure 3, the looped portion 108 of the expanded medical device 110 may be located in the capture element 106. Sheath 114 is located over the capture element 106. The outer diameter of the proximal end 104 of the delivery tool may be larger than the outer diameter of the shaft 102 of the delivery tool. The sheath 114 may fit closely around or contact the proximal end 104 of the delivery tool and may fit less closely around the shaft 102 of the delivery tool. The inner diameter of the sheath 114 may be similar or equal to the outer diameter of the proximal end 104 of the delivery tool such that the sheath 114 prevents the looped portion 108 from leaving the capture element 106. The space shown between the sheath 114 and proximal end 104 of the delivery tool in Figures 1 - 4 is exaggerated to show the components of the exemplary medical device delivery system 100 more clearly. While the sheath 114 is located over the capture element 106, the sheath 114 prevents the looped portion 108 of the medical device 110 from leaving the capture element 106. The close fit of the sheath 114 over the proximal end 104 of the delivery tool may help prevent the looped portion 108 of the medical device 110 from leaving the capture element 106.

The capture element 106 may be shaped to prevent the looped portion 108 of the medical device 110 from leaving the capture element 106. The capture element 106 may consist of a groove, notch, or other indentation in the proximal end 104 of the delivery tool. The depth, width, and shape of the capture element 106 may be adjusted based on the size of the looped portion 108 of the medical device 110. The capture element may be sized to allow a portion of the diameter of the looped portion 108 to be within the capture element 106 such that the diameter of the looped portion 108 will not increase the overall diameter of the delivery tool and sheath 114. The capture element may be located and shaped to allow the looped portion 108 to enter the capture element 106 before the proximal end 104 of the delivery tool reaches its maximum diameter. Allowing the looped portion 108 to enter the capture element 106 before the proximal end 104 of the delivery tool reaches its maximum diameter will enable the sheath 114 to have the smallest possible diameter because the sheath 114 diameter will be based on the maximum diameter of the delivery tool and not the looped portion 108 of the medical device 110 in addition to the delivery tool.

The capture element 106 may also be shaped to prevent the looped portion 108 of the medical device 110 from leaving the capture element 106 except by axial movement of the capture element 106 and shaft 102 towards the medical device 110. Rotation of the capture element 106 around the longitudinal axis of shaft 102 may be necessary to release the looped portion 108 from the capture element 106. The capture element 106 may include a protrusion 112 located at the proximal end of capture element 106. The protrusion 112 may extend over the looped portion 108 when looped portion 108 is located within capture element 106. The protrusion 112 may inhibit movement of the looped portion 108 that is substantially perpendicular to the longitudinal axis of shaft 102. The protrusion 112 may inhibit the looped portion 108 of the medical device 110 from leaving the capture element 106. Alternatively, the capture element 106 may not include a protrusion 112.

The capture element 106 may include an inclined surface 118 located on the distal end of capture element 106. Inclined surface 118 may aid the release of looped portion 108 from capture element 106 by guiding the looped portion 108 out of the capture element 106 when the looped portion 108 comes in contact with inclined surface 118. After the sheath 114 has been withdrawn, axial movement of the delivery tool toward medical device 110 may cause the looped portion 108 to contact the inclined surface 118. Inclined surface 118 may extend from the deepest level of capture element 106 to the outer diameter of the proximal end 104 of the delivery tool. Alternatively, the capture element 106 may not include an inclined surface 118.

Figure 4 is a side view of the exemplary medical device delivery system 100. In Figure 4, the sheath 114 has been withdrawn, i.e., moved axially away from the capture element 106. Moving the sheath 114 away from the capture element 106 exposes capture element 106. After sheath 114 is moved away from capture element 106 and capture element 106 is exposed, looped portion 108 may be released from capture element 106. The shape of capture element 106 may require movement of the delivery tool before the looped portion 108 of the medical device 110 is released from the capture element 106. For example, the protrusion 112 may inhibit the looped portion 108 from leaving the capture element 106 even when sheath 114 has been moved away from capture element 106. If this is the case, the delivery tool may need to be moved axially toward the medical device 110 to allow the looped portion 108 to disengage the protrusion 112 of capture element 106. After looped portion 108 is released from the capture element 106, the delivery tool may be removed and the previously expanded medical device 110 may remain in place at the desired location.

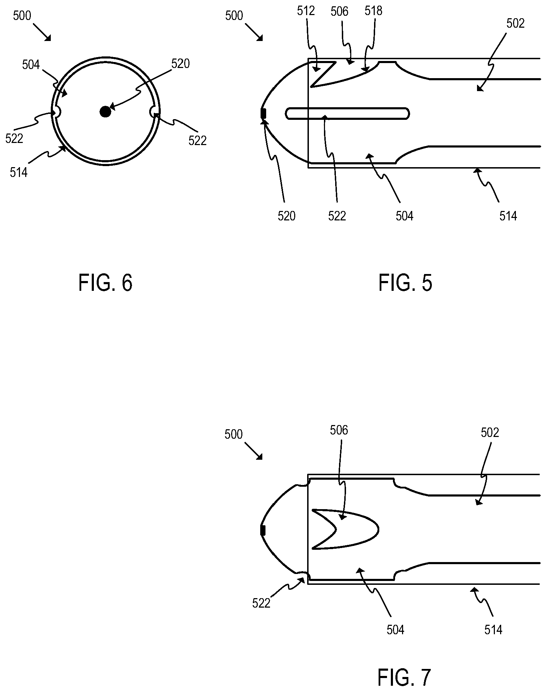

Figure 5 is a side view of portions of another exemplary medical device delivery system 500. Figure 5 may include a delivery tool with a distal end (not shown), a shaft 502, and a proximal end 504. The exemplary medical device delivery system 500 may also include components previous described in reference to exemplary medical device delivery system 100 but not shown in Figures 5 - 7, such as an expanded medical device located distally from the delivery tool and a guidewire or cannula. The medical device includes a looped portion at the distal end of the medical device, as previously described.

A sheath 514 surrounds the shaft 502 and proximal end 504 of the delivery tool in exemplary medical device delivery system 500. In Figures 5 and 7, the sheath 514 is shown as transparent to allow objects covered by the sheath 514 to be visible. The sheath 514 may actually be opaque or transparent. The sheath 514 may move axially relative to the shaft 502. The outer diameter of the proximal end 504 of the delivery tool may be larger than the outer diameter of the shaft 502 of the delivery tool. The sheath 514 may fit closely around or contact the proximal end 504 of the delivery tool and may fit less closely around the shaft 502 of the delivery tool. The inner diameter of the sheath 514 may be similar or equal to the outer diameter of the proximal end 504 of the delivery tool. The space shown between the sheath 514 and proximal end 504 of the delivery tool in Figures 5 - 7 is exaggerated to show the components of the exemplary medical device delivery system 500 more clearly.

Exemplary medical device delivery system 500 includes a capture element 506 at the proximal end 504 of the delivery tool. The looped portion of the medical device (not shown) is held in the capture element 506. The capture element 506 may be any shape capable of allowing a looped portion of a medical device to enter and be retained in the capture element 506. As previously described in reference to exemplary medical device delivery system 100, the capture element 506 may include a protrusion 512 and an inclined surface 518. The protrusion 512 may inhibit a looped portion of a medical device from leaving the capture element 506 in a direction substantially perpendicular to the longitudinal axis of shaft 502. Inclined surface 518 may aid the release of a looped portion from capture element 506 by guiding a looped portion out of the capture element 506 when a looped portion comes in contact with inclined surface 518.

As previously described in reference to exemplary medical device delivery system 100, the capture element 506 may be located and shaped to allow a sheath 514 covering the capture element 506 containing a looped portion of a medical device to be the smallest possible diameter because the sheath 514 diameter will be based on the maximum diameter of the delivery tool and not the delivery tool plus the looped portion of the medical device. The capture element 506 may allow a looped portion of the medical device to enter the capture element 506 before the proximal end 504 of the delivery tool reaches its maximum diameter.

Exemplary medical device delivery system 500 may include one or more channels 522 located in the proximal end 504 of the delivery tool to allow fluid to move from one side of the capture element 506 to another side of the capture element 506 while the sheath 514 is located over the capture element 506. The fluid may be used, for example, to flush the medical device located proximal to the delivery tool. The channels 522 may extend from one side of the capture element 506 to another side of the capture element 506. The channels 522 may be located along the perimeter of the proximal end 504 of the delivery tool, as shown in Figures 5 and 6, or may be located within the proximal end 504 of the delivery tool. If exemplary medical device delivery system 500 includes more than one channel 522, the channels 522 may be spaced regularly around the proximal end 504 of the delivery tool, as shown in Figures 5 and 6, or may be spaced irregularly to accommodate the shape of the proximal end 504 of the delivery tool or capture element 506.

Figure 6 is a front view of portions of exemplary medical device delivery system 500. The exemplary delivery tool shown in Figure 6 may be generally cylindrical or any other elongated shape capable of entering a body cavity. The delivery tool may include a lumen 520 provided through the shaft 502. The lumen 520 may allow a guidewire or cannula to pass axially through the delivery tool.

The channels 522 to transfer fluid from one side of the capture element 506 to another side of the capture element 506 while the sheath 514 is located over the capture element 506 may be hemispherical in shape, as shown in Figure 6, or may be any other shape capable of allowing fluid movement within the channel 522. The channels 522 may be sized to allow sufficient fluid, such as saline, to be transferred from one side of the capture element 506 to another side of the capture element 506, while the sheath 514 is covering the capture element 506, to flush a medical device located proximally from the delivery tool. The fluid used to flush a medical device located proximally from the delivery tool may originate at the distal end of the delivery tool and travel around the shaft 502 of the delivery tool underneath the sheath 514 covering the shaft 502.

Figure 7 is a top view of portions of exemplary medical device delivery system 500. Capture element 506 may occupy a portion of the width of the proximal end 504 of the delivery tool, as shown in Figure 7, or may extend the across the entire width of the delivery tool, as shown in Figure 1.

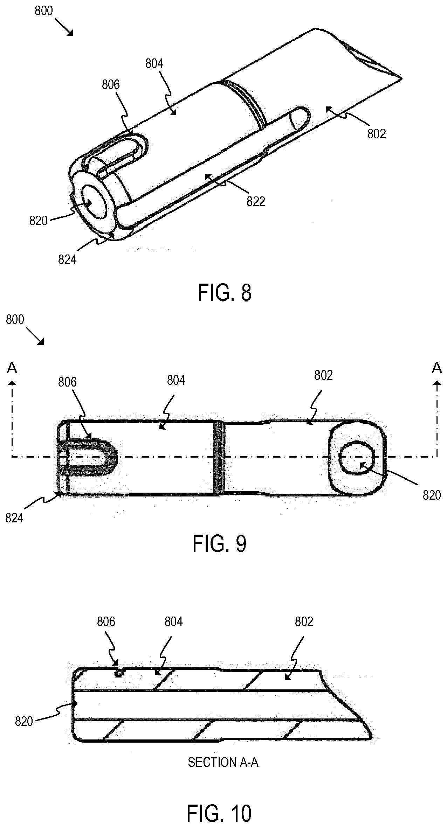

Figure 8 is a perspective view of portions of another exemplary medical device delivery system 800. Figure 8 may include a delivery tool with a distal end (not shown), a shaft 802, and a proximal end 804. The exemplary medical device delivery system 800 also includes components previous described in reference to exemplary medical device delivery systems 100 and 500 but not shown in Figures 8 - 10, such as an expanded medical device located proximally from the delivery tool, a guidewire or cannula, and a sheath covering the delivery tool. The medical device includes a looped portion at the distal end of the medical device, as previously described.

Exemplary medical device delivery system 800 includes a capture element 806 at the proximal end 804 of the delivery tool. The looped portion of the medical device (not shown) enters capture element 806 and be held in capture element 806 when a sheath covers capture element 806. The capture element 806 may be any shape capable of allowing a looped portion of a medical device to enter and be retained in the capture element 806. In exemplary medical device delivery system 800, the capture element 806 may consist of a "U" shaped recessed region in the proximal end 804 of the delivery tool. The depth and width of the recessed region may be sized to allow a looped portion of a medical device to completely enter capture element 806 and be covered by a sheath (not shown). The inner diameter of the sheath may be substantially similar or equal to the outer diameter of the proximal end 804 of the delivery tool. A close fit of a sheath over the capture element 806 and the proximal end 804 of the delivery tool may prevent a looped portion of a medical device from being released from the capture element 806.

The open end of the "U" shaped capture element 806 may extend to the extreme proximal end 804 of the delivery tool. Extending the capture element 806 to the extreme proximal end 804 of the delivery tool may allow looped portion of the medical device to enter the capture element from the extreme proximal end 804 of the delivery tool, which may allow the delivery tool and sheath to have a smaller combined diameter and lower profile because the width of the looped portion will not add to the diameter of the delivery tool and sheath. The extreme proximal end 804 of the delivery tool may include beveled or rounded edges 824. Exemplary medical device delivery system 800 may also include channels 822. As previously described in reference to exemplary medical device delivery system 500, the channels 822 may allow fluid to move from one side of the capture element 806 to another side of the capture element 806 while a sheath (not shown) is located over the capture element 806. Channels 822 may be located and sized to accommodate the particular need to transfer fluid from one side of the capture element 806 to another side of the capture element 806, such as to flush a stent-graft medical device located proximally from the delivery tool.

Figure 9 is a top view of portions of exemplary medical device delivery system 800. In Figure 9, the shaft 802 of the delivery tool has been cut away to expose the lumen 820. Lumen 820 may extend from the proximal end of the delivery tool to the distal end of the delivery tool. The lumen 820 may allow a guidewire or cannula to pass axially through the delivery tool. The guidewire or cannula may extend beyond the delivery tool through a medical device located distally from the delivery tool.

Figure 10 is a side sectional view of portions of exemplary medical device delivery system 800. In Figure 10, the shaft 802 and proximal end 804 of the delivery tool have been cut away along section line A-A in Figure 9 to expose the lumen 820 and capture element 806. The segment of the capture element 806 shown in Figure 10 may be located along the curved part of the "U" shaped capture element 806. The capture element 806 may be sized to accommodate the looped portion of a medical device.

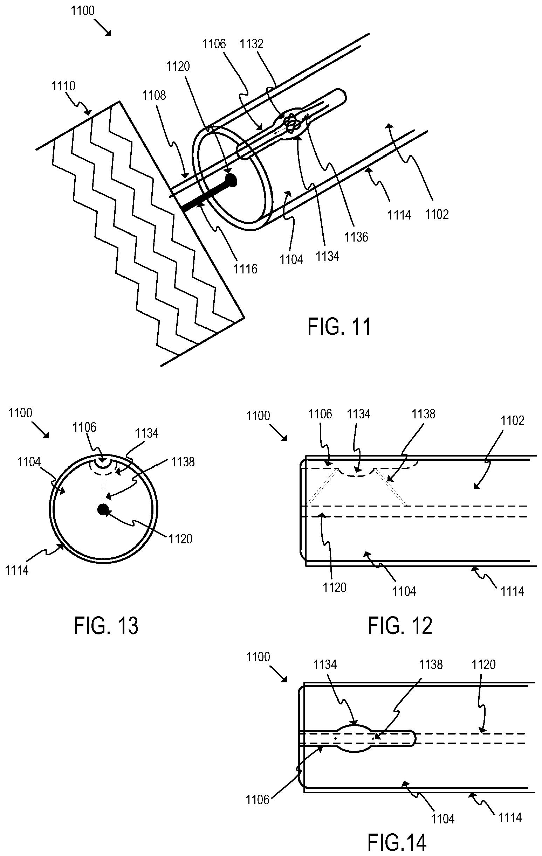

Figure 11 is a perspective view of another exemplary medical device delivery system 1100. Figure 11 may include a delivery tool with a distal end (not shown), a shaft 1102, and a proximal end 1104. The delivery tool may include a lumen 1120 provided through the shaft 1102. The lumen 1120 may allow a guidewire or cannula 1116 to pass axially through the delivery tool.

A medical device 1110 may be located proximally from the proximal end 1104 of the delivery tool. The medical device 1110 may be a stent-graft or any other medical device designed to be located intravascularly or within a body cavity. The medical device 1110 in Figure 11 is shown in an expanded state. As previously discussed in reference to delivery system 100, a sheath 1114 may have previously radially restrained the medical device 1110 while the sheath 1114 was coaxially located over the medical device 1110. Removal of sheath 1114 may have allowed medical device 1110 to expand radially, as shown in Figure 11. As previously discussed in reference to delivery system 100, the medical device 1110 includes a looped portion 1108 located at its distal end. The looped portion 1108 may be any resilient and flexible structure, such as a suture, attached to the medical device 1110. The looped portion 1108 may include one or more free ends. The looped portion 1108 may include an enlarged section 1132, such as a knot, twist, or bend. The enlarged section 1132 may be created by tying or twisting together free ends of the looped portion 1108. The enlarged section 1132 may also include a structure to increase the size of the enlarged section 1132. The structure may include, for example, a polymer ball attached to the looped portion 1108. The enlarged section 1132 may be created by any other means known to those of ordinary skill in the art.

Sheath 1114 surrounds the shaft 1102 and proximal end 1104 of the delivery tool in exemplary medical device delivery system 1100. In Figures 11, 12, and 14, the sheath 1114 is shown as transparent to allow objects covered by the sheath 1114 to be visible. The sheath 1114 may actually be opaque or transparent. The sheath 1114 may move axially relative to the shaft 1102. The sheath 1114 may fit closely around or contact the proximal end 1104 of the delivery tool and may fit less closely around the shaft 1102 of the delivery tool. The inner diameter of the sheath 1114 may be similar or equal to the outer diameter of the proximal end 1104 of the delivery tool. The space shown between the sheath 1114 and proximal end 1104 of the delivery tool in Figures 11 - 14 is exaggerated to show the components of the exemplary medical device delivery system 1100 more clearly.

Exemplary medical device delivery system 1100 includes a capture element 1106 at the proximal end 1104 of the delivery tool. The capture element 1106 may consist of a groove, notch, or other indentation in the proximal end 1104 of the delivery tool. The capture element 1106 may include a cavity 1134 designed to contain the enlarged section 1132 of the looped portion 1108. The depth, width, and shape of the capture element 1106 and cavity 1134 may be adjusted based on the size of the looped portion 1108 and enlarged section 1132 of the medical device 1110. The capture element 1106 may be sized to allow the looped portion 1108 and enlarged section 1132 to be within the capture element 1106 such that the looped portion 1108 will not increase the overall diameter of the delivery tool and sheath 1114. The looped portion 1108 of the medical device may be held in the capture element 1106. The enlarged section 1132 of the looped portion 1108 may fit into a cavity 1134 of the capture element. While the sheath 1114 is located over the capture element 1106, the sheath 1114 may prevent the enlarged section 1132 from leaving the capture element 1106. The enlarged section 1132 may be sized to be larger than the space between the sheath 1114 and the proximal end 1104 of the delivery tool such that the looped portion 1108 may be prevented from leaving the capture element 1106 while the sheath 1114 is located over the capture element 1106. The close fit of the sheath 1114 over the proximal end 1104 of the delivery tool may help prevent the enlarged section 1132 from leaving the capture element 1106.

Capture element 1106 may extend to the extreme proximal end 1104 of the delivery tool, as shown in Figures 11 - 14. Extending the capture element 1106 to the extreme proximal end 1104 of the delivery tool may allow looped portion 1108 of the medical device to enter the capture element 1106 from the extreme proximal end 1104 of the delivery tool, which may allow the delivery tool and sheath 1114 to have a smaller combined diameter and lower profile because the looped portion 1108 will not add to the diameter of the delivery tool and sheath 1114.

Delivery system 1100 may include a trigger wire 1136 to facilitate easier loading of the medical device 1110. Trigger wire 1136 may hold enlarged section 1132 in cavity 1134 while sheath 1114 is placed over the looped portion 1108 and capture element 1106. Trigger wire 1136 may be extended from the distal end (not shown) of the delivery tool through lumen 1120 to two holes 1138 that are located on two sides of cavity 1134. Holes 1138 may extend from lumen 1120 at an angle to prevent the trigger wire 1136 from extending through sharp bends. Enlarged section 1132 may be placed in cavity 1134 during loading of the medical device 1110. Next, trigger wire 1136 may be threaded up from lumen 1120 through one of holes 1138 and placed over enlarged section 1132 and then threaded down through the other hole 1138, as shown in Figure 11. Trigger wire 1136 may be removed after sheath 1114 is placed over the looped portion 1108 and capture element 1106 because sheath 1114 may prevent the enlarged section 1132 from leaving the capture element 1106. Trigger wire 1136 may be removed before delivery system 1100 is delivered to a location in a body.

Figure 12 is a side view of portions of another exemplary medical device delivery system 1100. Figure 12 shows capture element 1106, cavity 1134, holes 1138, and lumen 1120 in shaded line. Figure 12 shows a possible depth of cavity 1134 into the proximal end 1104 of the delivery tool.

Figure 13 is a front view of portions of exemplary medical device delivery system 1100. Figure 13 shows cavity 1134 and holes 1138 in shaded line. Figure 13 shows that cavity 1134 may not extend to the extreme proximal end 1104 of the delivery tool.

Figure 14 is a top view of portions of exemplary medical device delivery system 1100. Figure 14 shows lumen 1120 in shaded line. Figure 14 shows a possible width of capture element 1106 and cavity 1134. Figure 14 also shows that cavity 1134 may not extend to the extreme proximal end 1104 of the delivery tool.

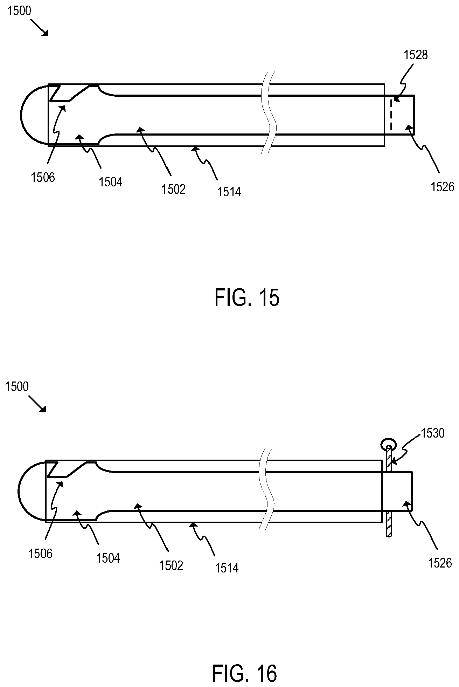

Figures 15 and 16 are side views of portions of another exemplary medical device delivery system 1500. Figures 15 and 16 may include a delivery tool with a distal end 1526, a shaft 1502, a proximal end 1504, and capture element 1506, and sheath 1514. The exemplary medical device delivery system 1500 may also include components previous described in reference to exemplary medical device delivery systems 100, 500, 800, or 1100 but not shown in Figures 15 and 16, such as an expanded medical device with a looped portion located proximally from the delivery tool and a guidewire or cannula. Exemplary medical device delivery system 1500 may also include a safety mechanism designed to prevent the unintentional uncovering of the capture element 1506 by movement of the sheath 1514. Unintentionally uncovering the capture element 1506 may inadvertently release the looped portion of the medical device from the capture element 1506, which may deploy the medical device prematurely. The safety mechanism may be located at the distal end 1526 of the delivery tool. For example, the safety mechanism shown in Figure 15 may consist of a mark 1528 on the delivery tool or sheath 1514 indicating the point at which moving the sheath 1514 distally will begin to uncover the capture element 1506. The mark 1528 may warn a physician using the delivery system that moving the sheath 1514 past the mark 1528 will uncover the capture element 1506 and deploy the medical device.

Figure 16 shows another exemplary safety mechanism to prevent inadvertently uncovering the capture element 1506. The safety mechanism in Figure 16 may include a structure to physically prevent uncovering the capture element. The structure may include a removable pin 1530 or stop at the distal end 1526 of the delivery tool that prevents the sheath 1514 from moving distally past a point in which the capture element 1506 would be uncovered. The removable pin 1530 may need to be moved before movement of the sheath 1514 can uncover the capture element 1506. The safety mechanism may also include radiopaque portions of the sheath, proximal end of the delivery tool, and capture element that can indicate the locations of the sheath, proximal end of the delivery tool, and capture element inside a body cavity to a physician. The physician may use the radiopaque portions to know when movement of the sheath will uncover the capture element and deploy the medical device. Any of the medical device delivery systems previously described may include a delivery tool with a safety mechanism designed to prevent the unintentional uncovering of the capture element by movement of the sheath.

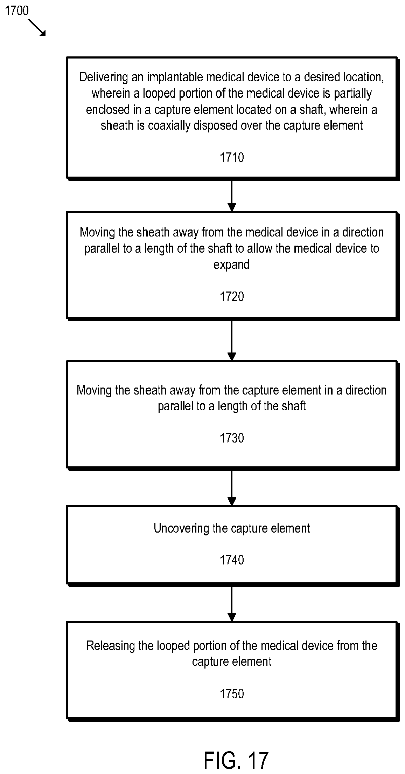

Figure 17 illustrates method 1700 for releasing a medical device. The method begins with step 1710 in which an implantable medical device is delivered to a desired location. The implantable medical device, such as a stent-graft, has a looped portion located on its distal end. The looped portion is partially enclosed in a capture element located on a shaft of a delivery tool that is distal to the medical device. A sheath is coaxially disposed over the medical device and capture element. The sheath radially restrains the medical device in a compressed state. Step 1720 involves moving the sheath away from the medical device in a direction parallel to a length of the shaft to uncover the medical device and allow the medical device to expand. Step 1730 involves moving the sheath away from the capture element in a direction that is parallel to the length of the shaft. Step 1740 involves uncovering the capture element by moving the sheath away from the capture element. Step 1750 involves releasing the looped portion of the medical device from the capture element, which releases the medical device from the delivery tool.

## Claims

1. A medical device delivery system (100, 500, 800, 1100, 1500) for use with an expandable medical device (110, 1110) delivered in a vessel and having a resiliently flexible looped portion (108, 1108) extending from a section of the expandable medical device (110, 1110), the system including:
a shaft (102, 502, 802, 1102, 1502) including a proximal end and a distal end and an interposed internal lumen (520, 820, 1120), the shaft (102, 502, 802, 1102, 1502) having a notched portion (106, 506, 806, 1106, 1506) at the proximal end of the shaft (102, 502, 802, 1102, 1502), wherein the notched portion (106, 506, 806, 1106, 1506) receives the looped portion (108, 1108) of the expandable medical device for capture; and
a sheath (114, 514, 1114, 1514) coaxially disposed over the shaft (102, 502, 802, 1102, 1502), wherein the sheath (114, 514, 1114, 1514) is movable from a first axial position where the sheath (114, 514, 1114, 1514) covers the notched portion (106, 506, 806, 1106, 1506) to a second axial position where the notched portion (106, 506, 806, 1106, 1506) is not covered by the sheath (114, 514, 1114, 1514) thereby releasing the looped portion from capture, wherein the expandable medical device is coupled to the shaft (102, 502, 802, 1102, 1502) when the sheath (106, 506, 806, 1106, 1506) is in the first axial position, **characterized in that**
the expandable medical device is a stent/stent-graft and the looped portion received by the notched portion extends from the distal section of the stent/stent-graft.

2. A system according to claim 1, wherein the notched portion (106, 506, 806, 1106, 1506) extends over the looped portion (108, 1108) of the stent/stent-graft (110, 1110) when the looped portion (108, 1108) of the stent/stent-graft (110, 1110) is in the notched portion (106, 506, 806, 1106, 1506).

3. A system according to claim 1 or 2, wherein an outer diameter of the shaft (102, 502, 802, 1102, 1502) in the region of the notched portion (106, 506, 806, 1106, 1506) is the same as an inner diameter of the sheath (114, 514, 1114, 1514).

4. A system according to any preceding claim, wherein the notched portion (106, 506, 806, 1106, 1506) includes a proximal end and an distal end and an interposed enlarged cavity (1134), wherein the enlarged cavity (1134) does not extend to an extreme proximal end of the shaft (102, 502, 802, 1102, 1502), and wherein the enlarged cavity (1134) receives an enlarged section (1132) of the looped portion (108, 1108) of the stent/stent-graft (110, 1110).

5. A system according to any preceding claim, including a channel (522, 822) located at the proximal end (104, 504, 804 1104, 1504) of the shaft (102, 502, 802, 1102, 1502), wherein the channel (522, 822) allows fluid to move from one side of the notched portion (106, 506, 806, 1106, 1506) to another side of the notched portion (106, 506, 806, 1106, 1506) while the sheath (114, 514, 1114, 1514) is in the first axial position.

## Patentansprüche

1. Medizinisches Vorrichtungszufuhrsystem (100, 500, 800, 1100, 1500) zur Verwendung mit einer expandierbaren medizinischen Vorrichtung (110, 1110), die in ein Gefäß zugeführt wird und einen federnd flexiblen Schlaufenabschnitt (108, 1108) hat, der sich von einer Sektion der expandierbaren medizinischen Vorrichtung (110, 1110) erstreckt, wobei das System Folgendes aufweist:
einen Schaft (102, 502, 802, 1102, 1502), der ein proximales Ende und ein distales Ende und ein dazwischenliegendes inneres Lumen (520, 820, 1120) aufweist, wobei der Schaft (102, 502, 802, 1102, 1502) einen eingekerbten Abschnitt (106, 506, 806, 1106, 1506) am proximalen Ende des Schafts (102, 502, 802, 1102, 1502) hat, wobei der eingekerbte Abschnitt (106, 506, 806, 1106, 1506) den Schlaufenabschnitt (108, 1108) der expandierbaren medizinischen Vorrichtung zum Festhalten aufnimmt, und
eine koaxial über dem Schaft (102, 502, 802, 1102, 1502) angeordnete Hülse (114, 514, 1114, 1514), wobei die Hülse (114, 514, 1114, 1514) aus einer ersten axialen Position, in der die Hülse (114, 514, 1114, 1514) den eingekerbten Abschnitt (106, 506, 806, 1106, 1506) abdeckt, in eine zweite axiale Position bewegbar ist, in der der eingekerbte Abschnitt (106, 506, 806, 1106, 1506) nicht von der Hülse (114, 514, 1114, 1514) abgedeckt ist, wodurch der festgehaltene Schlaufenabschnitt freigegeben wird, wobei die expandierbare medizinische Vorrichtung an den Schaft (102, 502, 802, 1102, 1502) gekoppelt ist, wenn die Hülse (106, 506, 806, 1106, 1506) in der ersten axialen Position ist, **dadurch gekennzeichnet, dass**
die expandierbare medizinische Vorrichtung ein Stent/Stentgraft ist und sich der vom eingekerbten Abschnitt aufgenommene Schlaufenabschnitt von der distalen Sektion des Stents/Stentgrafts erstreckt.

2. System nach Anspruch 1, wobei sich der eingekerbte Abschnitt (106, 506, 806, 1106, 1506) über den Schlaufenabschnitt (108, 1108) des Stents/Stentgrafts (110, 1110) erstreckt, wenn der Schlaufenabschnitt (108, 1108) des Stents/Stentgrafts (110, 1110) im eingekerbten Abschnitt (106, 506, 806, 1106, 1506) ist.

3. System nach Anspruch 1 oder 2, wobei ein äußerer Durchmesser des Schafts (102, 502, 802, 1102, 1502) im Bereich des eingekerbten Abschnitts (106, 506, 806, 1106, 1506) derselbe wie ein innerer Durchmesser der Hülse (114, 514, 1114, 1514) ist.

4. System nach einem der vorhergehenden Ansprüche, wobei der eingekerbte Abschnitt (106, 506, 806, 1106, 1506) ein proximales Ende und ein distales Ende und einen dazwischenliegenden vergrößerten Hohlraum (1134) aufweist, wobei sich der vergrößerte Hohlraum (1134) nicht zu einem äußersten proximalen Ende des Schafts (102, 502, 802, 1102, 1502) erstreckt und wobei der vergrößerte Hohlraum (1134) eine vergrößerte Sektion (1132) des Schlaufenabschnitts (108, 1108) des Stents/Stentgrafts (110, 1110) aufnimmt.

5. System nach einem der vorhergehenden Ansprüche, das einen Kanal (522, 822) aufweist, der am proximalen Ende (104, 504, 804, 1104, 1504) des Schafts (102, 502, 802, 1102, 1502) angeordnet ist, wobei der Kanal (522, 822) gestattet, dass sich Fluid von einer Seite des eingekerbten Abschnitts (106, 506, 806, 1106, 1506) zu einer anderen Seite des eingekerbten Abschnitts (106, 506, 806, 1106, 1506) bewegt, während die Hülse (114, 514, 1114, 1514) in der ersten axialen Position ist.

## Revendications

1. Système de pose (100, 500, 800, 1100, 1500) de dispositif médical destiné à être utilisé avec un dispositif médical déployable (110, 1110) posé dans un vaisseau et possédant une partie en boucle (108, 1108) flexible de façon résiliente s'étendant depuis une section du dispositif médical déployable (110, 1110), le système comprenant :
un arbre (102, 502, 802, 1102, 1502) comprenant une extrémité proximale et une extrémité distale et une lumière interne interposée (520, 820, 1120), l'arbre (102, 502, 802, 1102, 1502) possédant une partie entaillée (106, 506, 806, 1106, 1506) au niveau de l'extrémité proximale de l'arbre (102, 502, 802, 1102, 1502), dans lequel la partie entaillée (106, 506, 806, 1106, 1506) reçoit la partie en boucle (108, 1108) du dispositif médical déployable pour une capture ; et
une gaine (114, 514, 1114, 1514) disposée de manière coaxiale sur l'arbre (102, 502, 802, 1102, 1502), dans lequel la gaine (114, 514, 1114, 1514) est mobile depuis une première position axiale où la gaine (114, 514, 1114, 1514) recouvre la partie entaillée (106, 506, 806, 1106, 1506) vers une seconde position axiale où la partie entaillée (106, 506, 806, 1106, 1506) n'est pas recouverte par la gaine (114, 514, 1114, 1514) ce qui permet de dégager la partie en boucle de la capture, dans lequel le dispositif médical déployable est accouplé à l'arbre (102, 502, 802, 1102, 1502) lorsque la gaine (106, 506, 806, 1106, 1506) est dans la première position axiale, **caractérisé en ce que**
le dispositif médical déployable est une endoprothèse/greffe endovasculaire et la partie en boucle reçue par la partie entaillée s'étend depuis la section distale de l'endoprothèse/la greffe endovasculaire.

2. Système de selon la revendication 1, dans lequel la partie entaillée (106, 506, 806, 1106, 1506) s'étend sur la partie en boucle (108, 1108) de l'endoprothèse/la greffe endovasculaire (110, 1110) lorsque la partie en boucle (108, 1108) de l'endoprothèse/la greffe endovasculaire (110, 1110) est dans la partie entaillée (106, 506, 806, 1106, 1506).

3. Système selon la revendication 1 ou 2, dans lequel un diamètre extérieur de l'arbre (102, 502, 802, 1102, 1502) dans la région de la partie entaillée (106, 506, 806, 1106, 1506) est identique à un diamètre intérieur de la gaine (114, 514, 1114, 1514).

4. Système selon l'une quelconque des revendications précédentes, dans lequel la partie entaillée (106, 506, 806, 1106, 1506) comprend une extrémité proximale et une extrémité distale et une cavité agrandie (1134) interposée, dans lequel la cavité agrandie (1134) ne s'étend pas vers une extrémité proximale extrême de l'arbre (102, 502, 802, 1102, 1502), et dans lequel la cavité agrandie (1134) reçoit une section agrandie (1132) de la partie en boucle (108, 1108) de l'endoprothèse/la greffe endovasculaire (110, 1110).

5. Système selon l'une quelconque des revendications précédentes, comprenant un canal (522, 822) situé au niveau de l'extrémité proximale (104, 504, 804, 1104, 1504) de l'arbre (102, 502, 802, 1102, 1502), dans lequel le canal (522, 822) permet qu'un fluide se déplace d'un côté de la partie entaillée (106, 506, 806, 1106, 1506) vers un autre côté de la partie entaillée (106, 506, 806, 1106, 1506) pendant que la gaine (114, 514, 1114, 1514) est dans la première position axiale.
